(19) Europäisches Patentamt
European Patent Office
Office européen des brevets

(11) Veröffentlichungsnummer: **0 226 028 A2**

(12) # EUROPÄISCHE PATENTANMELDUNG

(21) Anmeldenummer: 86115432.6

(22) Anmeldetag: 07.11.86

(51) Int.Cl.⁴: **C 07 C 49/16**
C 07 C 45/63, C 07 C 49/255
C 07 C 45/71, C 07 D 249/08

(30) Priorität: 19.11.85 US 799540

(43) Veröffentlichungstag der Anmeldung:
24.06.87 Patentblatt 87/26

(84) Benannte Vertragsstaaten:
BE CH DE FR GB IT LI NL

(71) Anmelder: MOBAY CORPORATION
Patent Department Mobay Road
Pittsburgh Pennsylvania 15205-9741(US)

(72) Erfinder: Jackman, Dennis E.
7350 Roe Circle
Prairie Village Kansas 66208(US)

(74) Vertreter: Schumacher, Günter, Dr. et al,
c/o Bayer AG Konzernverwaltung RP Patentabteilung
D-5090 Leverkusen 1 Bayerwerk(DE)

(54) **Verfahren zur Herstellung von Monochlorpinakolon.**

(57) Die Erfindung betrifft ein neues Verfahren zur Herstellung von Monochlorpinakolon der Formel

$$CH_3-\overset{\displaystyle CH_3}{\underset{\displaystyle CH_3}{\overset{|}{\underset{|}{C}}}}-CO-CH_2-Cl$$

durch Umsetzung von Pinakolon mit Chlor in Gegenwart eines Chlorwasserstoff enthaltenden Lösungsmittels. Das entstehende Monochlorpinakolon enthält das Nebenprodukt Dichlorpinakolon nur in sehr geringer Menge.

EP 0 226 028 A2

Verfahren zur Herstellung von Monochlorpinakolon

Die vorliegende Erfindung betrifft ein neues Verfahren zur Herstellung von Monochlorpinakolon durch Umsetzung von Pinakolon mit Chlor in Gegenwart eines Chlorwasserstoff enthaltenden Lösungsmittels.

Die DE-OS 28 19 264 offenbart die folgende Synthese, die vom Pinakolon ausgeht und bei dem bekannten Herbizid N,N-Dimethyl-N'-(5-t-butyl-oxazol-3-yl)-harnstoff endet:

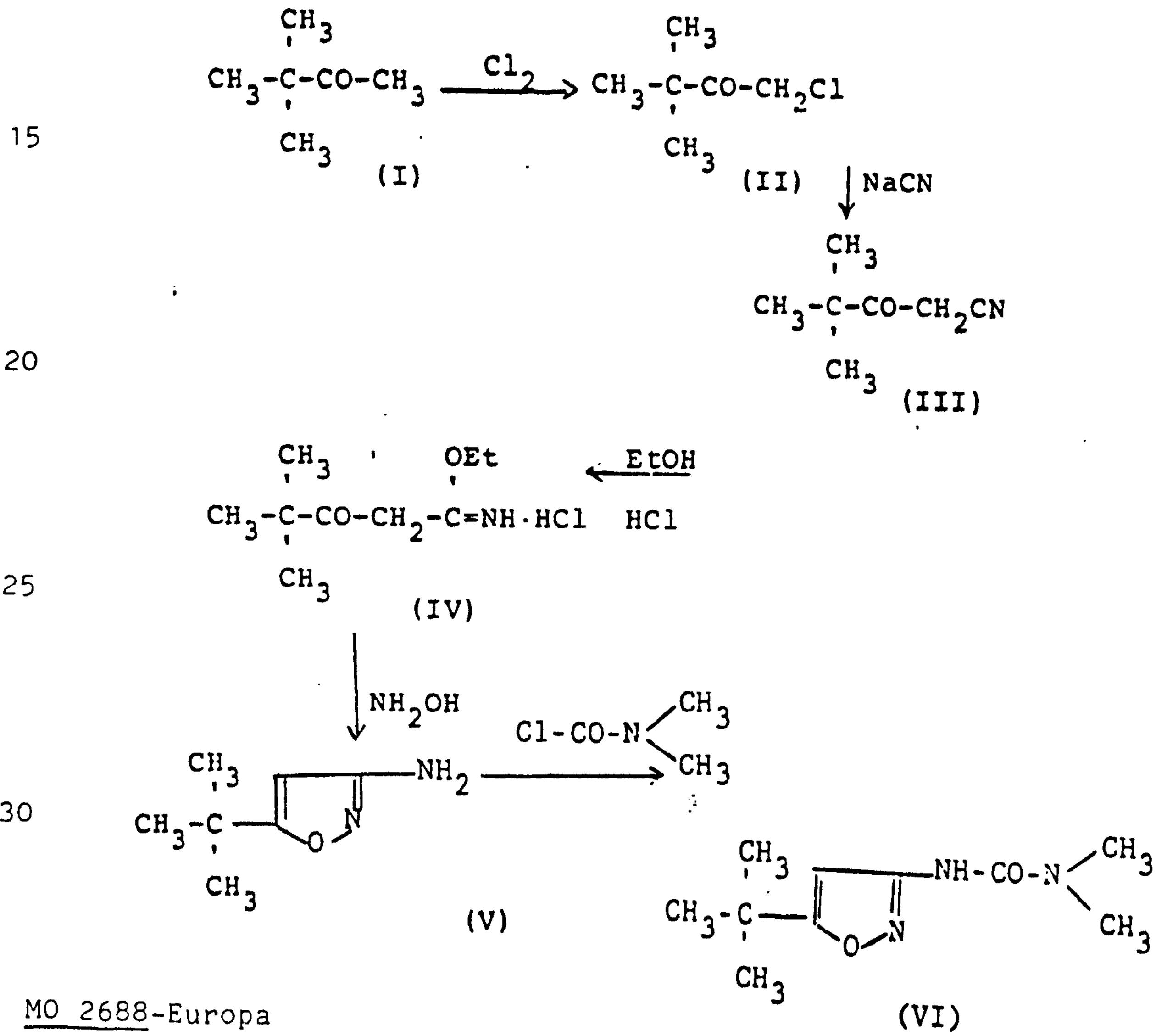


(I) (II) (III) (IV) (V) (VI)

Beim Arbeiten nach diesem Verfahren erhält man das Monochlorpinakolon der Formel (II) in einer hohen Ausbeute (92 % der Theorie). Nachteilig ist aber, daß der Dichlorpinakolon-Gehalt des entstehenden Produktes mit etwa 4 % relativ hoch ist. Da es sich bei diesem Dichlorpinakolon um ein störendes Produkt handelt, das im nächsten Schritt der Synthese zu unerwünschten Nebenreaktionen führt, ist jeweils eine Destillation des Monochlorpinakolons erforderlich. Durch diese Maßnahme läßt sich zwar der Gehalt an Dichlorpinakolon auf 0,3 % senken, jedoch ist das Verfahren aufwendig und kostspielig.

Aus der US-PS 3 912 752 sind folgende Umsetzungen bekannt:

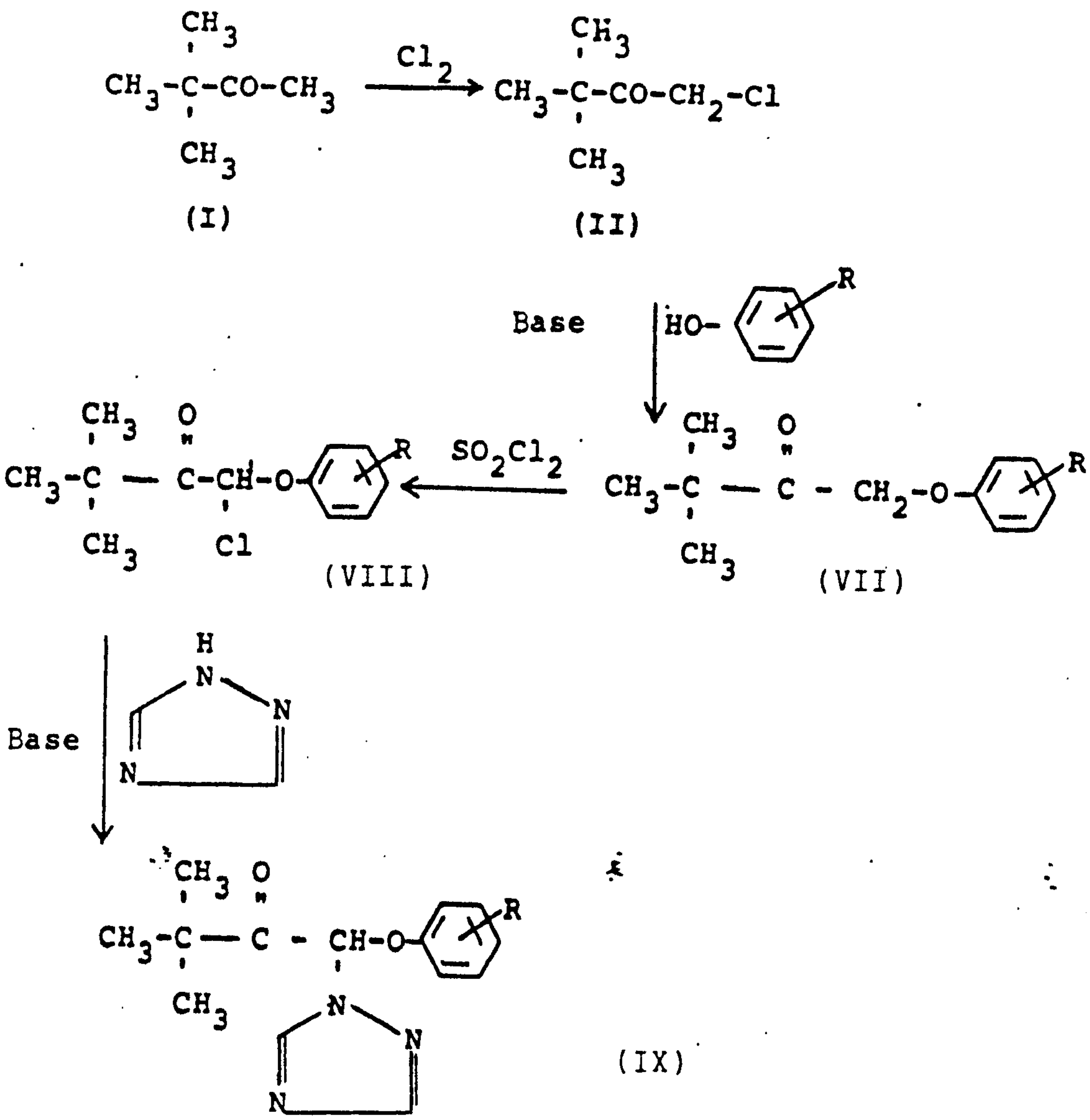


In den obigen Formeln steht

R vorzugsweise für Halogen, Phenyl, Phenoxy, Halogenphenyl und Halogenphenoxy.

Darüber hinaus kann R auch noch weitere Bedeutungen haben..

Die Verbindungen der Formel (IX) sind bekannte Fungizide.

Aus den Verbindungen der Formel (IX) lassen sich durch Reduktion der Keto-Gruppe die entsprechenden Carbinole der Formel

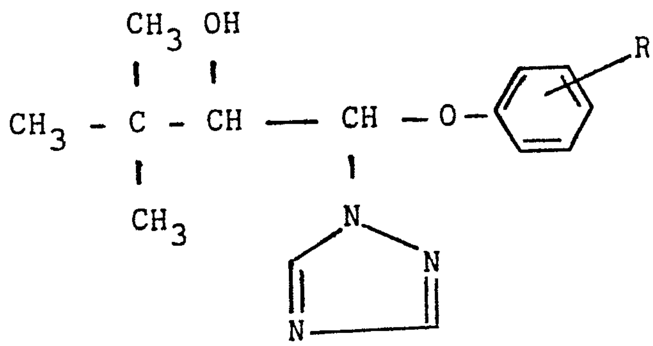

(X)

herstellen, die ebenfalls als Fungizide bekannt sind.

Auch bei der oben skizzierten Umsetzung ist ein Gehalt von Dichlorpinakolon in dem als Zwischenprodukt auftretenden Monochlorpinakolon nachteilig, denn dadurch werden die Ausbeuten in den anschließenden Reaktionsschritten erniedrigt. Da Dichlorpinakolon zwei Chloratome enthält, - im Vergleich zu nur einem Chloratom im Monochlorpinakolon-, hat die Anwesenheit von Dichlorpinakolon im Reaktionsgemisch tatsächlich sogar eine proportional höhere Einbuße an gewünschtem Produkt im nächsten Schritt der Synthese zur Folge. Setzt man zum Beispiel Monochlorpinakolon mit einem Gehalt von 4 % Dichlorpinakolon ein, so ergibt sich in der folgenden Stufe ein Produkt, das 8 % des unerwünschten Bis-Reaktionsproduktes enthalten kann. Die Abtrennung dieses Nebenproduktes ist schwierig. Aus diesem Grunde wandert es auch durch die weiteren Stufen und verunreinigt dann das Endprodukt.

Es ist ein Ziel der vorliegenden Erfindung, Monochlorpinakolon in solcher Reinheit zu erzeugen, daß es direkt für die weitere Synthese eingesetzt werden kann, so daß ohne vorherige Abtrennung von Verunreinigungen aus dem Monochlorpinakolon die Folgeprodukte in hohen Ausbeuten hergestellt werden können.

Ein Nebenprodukt der Chlorierung von Pinakolon ist HCl, das in dem Lösungsmittel gelöst verbleibt, in dem die Chlorierung gewöhnlich durchgeführt wird, z.B. einem Alkanol wie Methanol. Bei der Destillation entweicht vor der Verdampfung des Monochlorpinakolons HCl, das man entweder entweichen läßt oder in Wasser oder einem anderen Lösungsmittel auffängt, das ein alkalisches Neutralisationsmittel enthalten kann. Das Reaktionslösungsmittel geht nach dem HCl und vor dem Monochlorpinakolon über. Das Dichlorpinakolon ist am wenigsten flüchtig und bleibt meistens als Destillationsrückstand zurück.

Es wurde nun gefunden, daß man Monochlorpinakolon der Formel

$$\begin{array}{c} \quad CH_3 \\ \quad | \\ CH_3 - C - CO - CH_2 - Cl \\ \quad | \\ \quad CH_3 \end{array} \qquad \text{(II)}$$

mit einem sehr niedrigen Gehalt an Dichlorpinakolon erhält, wenn man Pinakolon der Formel

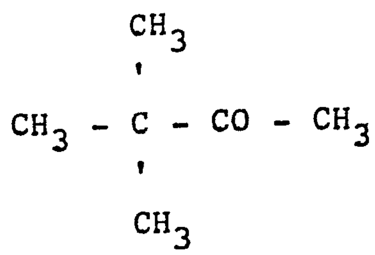

(I)

mit Chlor in Gegenwart eines Chlorwasserstoff enthaltenden Lösungsmittels umsetzt.

Es ist als ausgesprochen überraschend zu bezeichnen, daß sich nach dem erfindungsgemäßen Verfahren Monochlorpinakolon mit einem sehr niedrigen Gehalt an Dichlorpinakolon herstellen läßt, denn aufgrund des bekannten Standes der Technik konnte nicht erwartet werden, daß beim Arbeiten in Anwesenheit von Chlorwasserstoff enthaltenden Solventien die Bildung von Dichlorpinakolon zurückgedrängt wird.

Das erfindungsgemäße Verfahren zeichnet sich durch eine Reihe von Vorteilen aus. So sind die zu verwendenden Ausgangskomponenten auch in größeren Mengen zugänglich und technisch handhabbar. Ferner bereitet die Durchführung der Umsetzung keinerlei Schwierigkeiten. Die Aufarbeitung des anfallenden Gemisches ist ebenfalls problemlos, denn die flüchtigen Komponenten lassen sich durch einfaches Erhitzen entfernen. Von besonderem Vorteil ist schließlich, daß hochreines Monochlorpinakolon entsteht, das ohne zusätzliche Reinigung für weitere Umsetzungen verwendet werden kann.

Im allgemeinen geht man bei der Durchführung des erfindungsgemäßen Verfahrens so vor, daß man zu Beginn der Umsetzung ein Lösungsmittel verwendet, das mit Chlorwasserstoff beladen ist. Nach beendeter Umsetzung wird dieses Reaktionslösungsmittel nach Entfernung aus dem Reaktionsgemisch

nicht übermäßig gereinigt, sondern für die nächste Umsetzung in einem solchen Zustand, in dem es noch beträchtliche Mengen an Chlorwasserstoff enthält, erneut verwendet. Auf diese Weise kann Pinakolon in Monochlorpinakolon umgewandelt werden, das zu weniger als 1 % durch das Nebenprodukt Dichlorpinakolon verunreinigt ist. Am Ende der Reaktion wird das Reaktionsgemisch zum Sieden gebracht, um Chlorwasserstoff und Lösungsmittel zu verdampfen, die dann in einem weiteren Cyclus wieder eingesetzt werden können. Dabei verbleibt ein Destillationsrückstand von hochreinem Monochlorpinakolon, das einen so geringen Gehalt an Dichlorpinakolon aufweist, daß die direkte Verwendung in weiteren Umsetzungen möglich ist.

Wie bereits erwähnt, kann man für die Startreaktion (den ersten Cyclus) einfach das Lösungsmittel mit Chlorwasserstoff beladen. Das gewünschte Ergebnis läßt sich erzielen, wenn etwa 100 Mol-% Chlorwasserstoff, bezogen auf Pinakolon, vorhanden sind. Vorzugsweise arbeitet man mit Reaktionsgemischen, in denen 500 bis 700 Mol-% Chlorwasserstoff, bezogen auf Pinakolon, zugegen sind. Darüber hinaus ist es auch möglich, höhere Mengen an Chlorwasserstoff bis zur Sättigung des Lösungsmittels mit Chlorwasserstoff einzusetzen. Da jeder Cyclus mehr Chlorwasserstoff produziert, wird schließlich die Sättigung des Lösungsmittels mit Chlorwasserstoff erreicht, wenn man von der Tatsache absieht, daß etwas Chlorwasserstoff zu Beginn der jeweiligen Lösungsmittel-Entfernung durch Destillation aus dem Lösungsmittel entzogen wird.

Für die Durchführung des erfindungsgemäßen Verfahrens kommen als Lösungsmittel alle für Chlorierungen geeigneten Solventien mit mäßiger Flüchtigkeit in Betracht. Vorzugsweise verwendbar sind niedere Alkanole, wie Methanol.

Das Verhältnis von Lösungsmittel zu Pinakolon ist nicht kritisch, und ein Gewichtsverhältnis von wenigstens 5 : 1 ist geeignet. Mehr Lösungsmittel ist zulässig, muß dann jedoch am Ende der Reaktion abgekocht werden.

Die Chlorierung erfolgt vorzugsweise unter Einsatz von Chlor-Gas aus einer Stahlflasche. Das Gas wird vorteilhafterweise in das HCl-haltige Lösungsmittel eingeleitet, in dem das Pinakolon vorher gelöst wurde.

Die Reaktionstemperaturen können bei der Durchführung des erfindungsgemäßen Verfahrens innerhalb eines bestimmten Bereiches variiert werden. Im allgemeinen arbeitet man bei Temperaturen zwischen $-20^{\circ}$ C und $+30^{\circ}$ C, vorzugsweise zwischen $-10^{\circ}$ C und $+20^{\circ}$ C.

Die Reaktion wird im allgemeinen chargenweise durchgeführt, obwohl man auch Pinakolon inkrementell entsprechend dem Verbrauch zusetzen kann. Die Reaktion ist etwas exotherm, und Kühlung ist nötig, um die gewünschte Temperatur einzuhalten. Wenn mehr als die äquimolare Menge Chlor angeboten wird, hat dies einfach die Erzeugung von Dichlorpinakolon, einem unerwünschten Nebenprodukt, zur Folge. Wenn weniger als die äquimolare Menge Chlor angeboten wird, ist noch unumgesetztes Pinakolon vorhanden, das, falls es nicht zurückgewonnen wird, verschwendet werden würde. Jedoch wird nahezu kein Dichlorpinakolon gebildet, so daß ein geringer Pinakolon-Überschuß bevorzugt wird.

Wie angegeben wurde, kann der Monochlorpinakolon-Destillationsrückstand direkt weiter umgesetzt werden. Außer für die oben genannten Synthesen eines herbiziden Oxazolylharnstoffs kann es anderweitig verwendet werden, wo immer Monochlorpinakolon benötigt wird.

So lassen sich Verbindungen der Formel

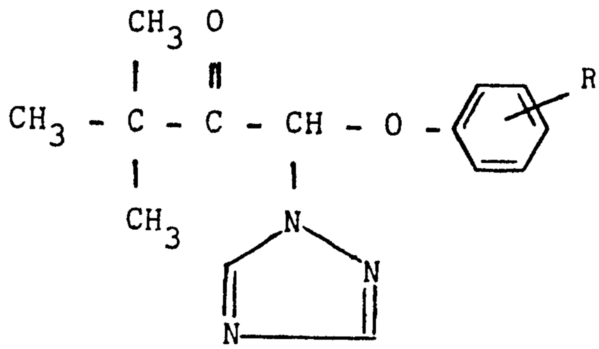

(IX)

in welcher

R für Halogen, Phenyl, Phenoxy, Halogenphenyl und Halogenphenoxy steht,

herstellen durch Reaktion von Pinakolon der Formel

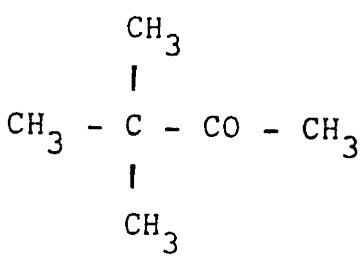

(I)

mit Chlor unter Bildung von Monochlorpinakolon der Formel

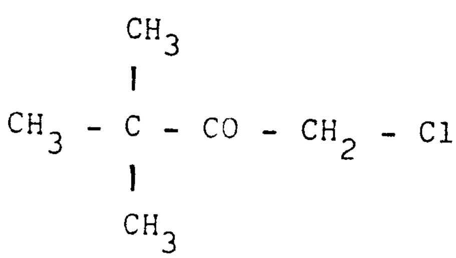

(II)

Umsetzung des Monochlorpinakolons der Formel (II) mit einem Phenol der Formel

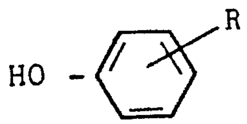

in welcher

R die oben angegebene Bedeutung hat,

Halogenierung des dabei entstehenden Phenoxy-pinakolon-Derivates der Formel

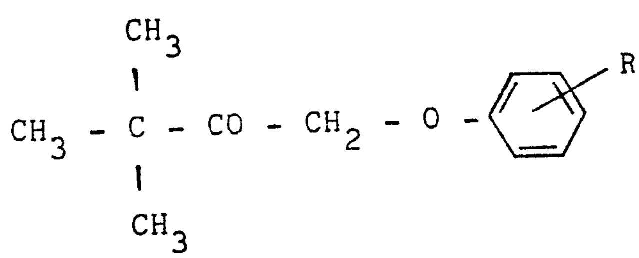

(VII)

in welcher

R die oben angegebene Bedeutung hat,

und Umsetzung des dabei anfallenden $\alpha$- Halogeno-$\alpha$-phenoxy-pinakolon-Derivates der Formel

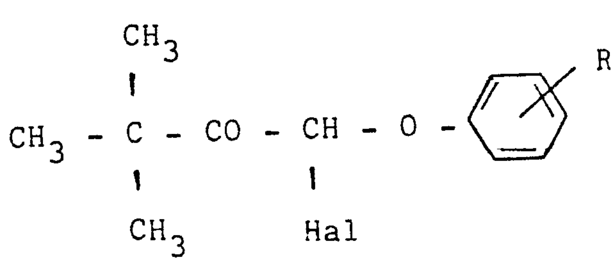

(VIII)

in welcher

R die oben angegebene Bedeutung hat und
Hal für Halogen steht,
mit 1,2,4-Triazol, wobei die Reaktion von Pinakolon mit Chlor in Gegenwart eines Chlrcwasserstoff enthaltenden Lösungsmittels durchgeführt wird und das Monochlorpinakolon direkt mit dem Phenol ohne vorherige Abtrennung von Dichlorpinakolon zur Reaktion gebracht wird.

Die Erfindung wird anhand der folgenden erläuternden Beispiele näher beschrieben, in denen alle Teile auf das Gewicht bezogen sind, sofern nichts anderes angegeben ist.

## Beispiel 1

102 g Pinakolon und 160 g Methanol wurden in einen Kolben gefüllt und auf -5° C gekühlt. HCl-Gas wurde bis zur Sättigung in den Kolbeninhalt eingeleitet, wobei die Lösung sich dunkelgelb färbte. 63 g Chlor wurden in die Lösung eingeleitet, die auf -10° C gehalten wurde. Etwa 10 min nach Beendigung der Einleitung des Chlors wurde die Kühlung unterbrochen und das Gefäß ausreichend erhitzt, um Methanol und überschüssiges Pinakolon über eine 20 cm-(8 inch) Vigreux-Kolonne abzudestillieren, wobei auch das HCl aus dem Kolben entfernt wurde. Der Destillationsrückstand umfaßte 0,8 g unumgesetztes Pinakolon, 118 g Monochlorpinakolon und 1,2 g Dichlorpinakolon, entsprechend 1,0 Gew.-% Dichlorpinakolon auf die vereinigten Gewichtsmengen Monochlorpinakolon und Dichlorpinakolon.

Das zurückgewonnene Methanol war mit HCl gesättigt und konnte danach in einem anderen Ansatz wiederverwendet werden.

## Beispiel 2

Eine Reihe von Ansätzen wurde im wesentlichen unter den gleichen Bedingungen mit wechselnden Mengen HCl, bezogen auf die Ausgangsmenge Pinakolon, durchgeführt.

Die Mengen der Ausgangsstoffe und Endprodukte sowie das Verhältnis des gewünschten Endprodukts zu dem unerwünschten Endprodukt sind in der nachstehenden Tabelle aufgeführt:

| Pinakolon g | HCl g | Gew.-% Chlor -Produkt Mono- | Di- | Gewichtsverhältnis Mono-/Di- |
|---|---|---|---|---|
| 100 | 56,6 | 95 | 3,0 | 33 |
| 100 | 113,2 | 95 | 1,5 | 64 |
| 100 | 226,4 | 95 | 1,1 | 85 |

## Beispiel 3

Stoffe, die wie in Beispiel 2 hergestellt worden waren, wurden in bekannter Weise entsprechend der Offenbarung der US-PS 3 912 752 eingesetzt, um das bekannte Fungizid 1-(4-Chlorphenoxy)-3,3-dimethyl-1-(1,2,4-triazol-1-yl)-butan-2-on auf dem folgenden Wege herzustellen:

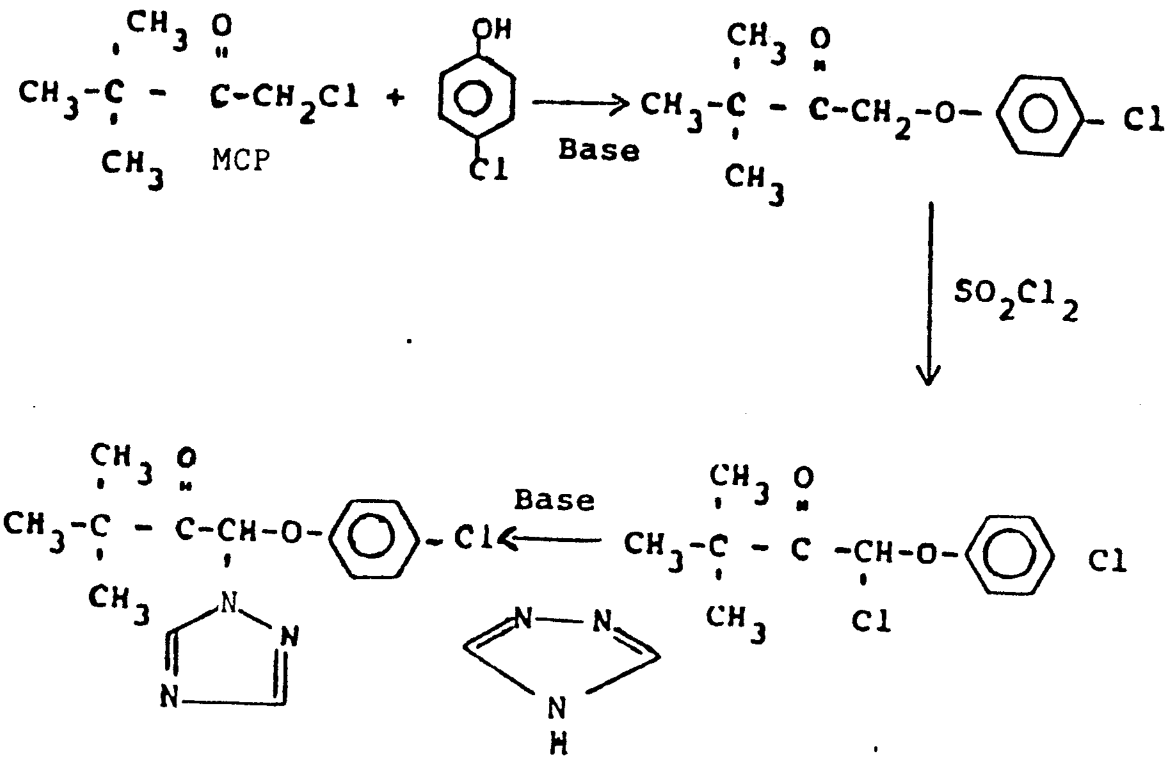


Die erhaltenen Ergebnisse waren folgende:

| Gew.-% Dichlor -Verbindung im Ausgangsstoff | 1 | 4 |
|---|---|---|
| % Wirkstoff im Produkt | 98,6 | 93,0 |
| % Netto-Ausbeute | 99,2 | 95,5 |

Patentansprüche

1. Verfahren zur Herstellung von Monochlorpinakolon der Formel

$$\begin{array}{ccccccccc} & & CH_3 & & & & & & \\ & & | & & & & & & \\ CH_3 & - & C & - & CO & - & CH_2 & - & Cl \\ & & | & & & & & & \\ & & CH_3 & & & & & & \end{array} \qquad (II)$$

mit einem sehr niedrigen Gehalt an Dichlorpinakolon, dadurch gekennzeichnet, daß man Pinakolon der Formel

$$\begin{array}{ccccccc} & & CH_3 & & & & \\ & & | & & & & \\ CH_3 & - & C & - & CO & - & CH_3 \\ & & | & & & & \\ & & CH_3 & & & & \end{array} \qquad (I)$$

mit Chlor in Gegenwart eines Chlorwasserstoff enthaltenden Lösungsmittels umsetzt.

2. Verfahren nach Anspruch 1, dadurch gekennzeichnet, daß am Ende der Reaktion das Lösungsmittel entfernt wird, wobei das Lösungsmittel das Nebenprodukt HCl enthält, und das HCl-haltige Lösungsmittel in einem weiteren Cyclus als Lösungsmittel eingesetzt wird.

3. Verfahren nach Anspruch 1, dadurch gekennzeichnet, daß HCl zu Beginn der Reaktion in einer Menge von wenigstens 500 Mol-%, bezogen auf Pinakolon, anwesend ist.

4. Verfahren nach Anspruch 1, dadurch gekennzeichnet, daß HCl zu Beginn der Reaktion in einer Menge von 500 bis 700 Mol-%, bezogen auf Pinakolon, anwesend ist.

5. Verfahren nach Anspruch 1, dadurch gekennzeichnet, daß das Lösungsmittel ein niederes Alkanol ist.

6. Verfahren nach Anspruch 1, dadurch gekennzeichnet, daß das Lösungsmittel Methanol ist.

7. Verfahren nach Anspruch 1, dadurch gekennzeichnet, daß das Lösungsmittel zu Beginn der Reaktion im wesentlichen mit HCl gesättigt ist.

8. Verfahren gemäß Anspruch 1, dadurch gekennzeichnet, daß man die Umsetzung bei Temperaturen zwischen $-20^{o}$ C und $+30^{o}$ C durchführt.

9. Verfahren zur Herstellung von Verbindungen der Formel

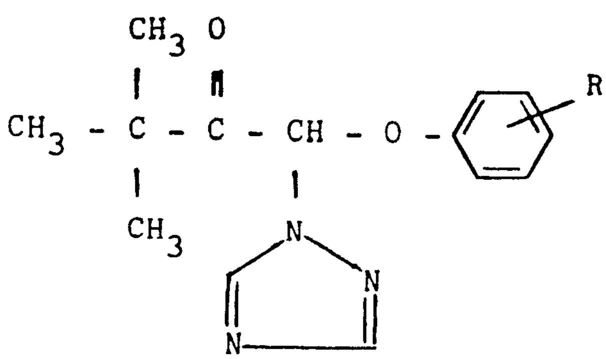

(IX)

in welcher

R für Halogen, Phenyl, Phenoxy, Halogenphenyl und Halogenphenoxy steht,

durch Reaktion von Pinakolon der Formel

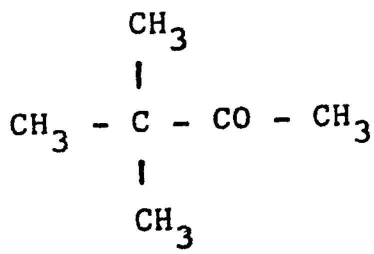

(I)

mit Chlor unter Bildung von Monochlorpinakolon der Formel

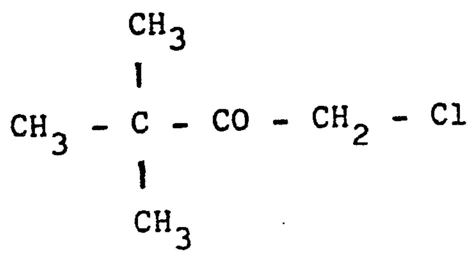

(II)

Umsetzung des Monochlorpinakolons der Formel (II) mit einem Phenol der Formel

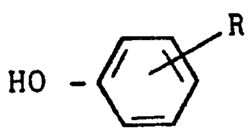

in welcher

R die oben angegebene Bedeutung hat,

Halogenierung des dabei entstehenden Phenoxy-pinakolon-Derivates der Formel

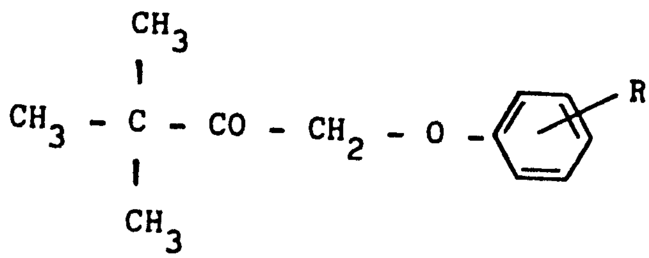

(VII)

in welcher

R die oben angegebene Bedeutung hat,

und Umsetzung des dabei anfallenden α-Halogeno-α-phenoxy-pinakolon-Derivates der Formel

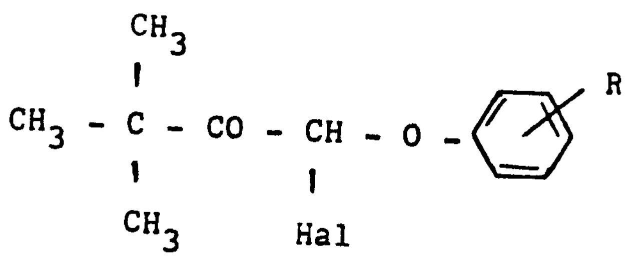

(VIII)

in welcher

R die oben angegebene Bedeutung hat und
Hal für Halogen steht,
mit 1,2,4-Triazol, dadurch gekennzeichnet, daß die Reaktion von Pinakolon mit Chlor in Gegenwart eines Chlorwasserstoff enthaltenden Lösungsmittels durchgeführt wird und das Monochlorpinakolon direkt mit dem Phenol ohne vorherige Abtrennung von Dichlorpinakolon zur Reaktion gebracht wird.